Europäisches Patentamt

European Patent Office

Office européen des brevets

(19)

(11) **EP 0 860 418 A1**

(12) **EUROPÄISCHE PATENTANMELDUNG**

(43) Veröffentlichungstag:
26.08.1998 Patentblatt 1998/35

(21) Anmeldenummer: 98102707.1

(22) Anmeldetag: 17.02.1998

(51) Int. Cl.$^6$: **C07C 45/36**, C07C 49/784,
C07C 49/786, C07C 2/66,
C07C 15/16

(84) Benannte Vertragsstaaten:
AT BE CH DE DK ES FI FR GB GR IE IT LI LU MC NL PT SE
Benannte Erstreckungsstaaten:
AL LT LV MK RO SI

(30) Priorität: 19.02.1997 DE 19706526

(71) Anmelder:
BASF AKTIENGESELLSCHAFT
67056 Ludwigshafen (DE)

(72) Erfinder:
• Gehrer, Eugen, Dr.
67069 Ludwigshafen (DE)

• Heidemann, Thomas, Dr.
69469 Weinheim (DE)
• Rust, Harald
67435 Neustadt (DE)
• Schäfer, Hans Dieter
67122 Altrip (DE)

(74) Vertreter:
Isenbruck, Günter, Dr. et al
Patent- und Rechtsanwälte Bardehle-
Pagenberg-Dost-Altenburg-Frohwitter-Geissler
& Partner
Theodor-Heuss-Anlage 12
68165 Mannheim (DE)

(54) **Verfahren zur Herstellung von aromatischen Ketonen**

(57) Es wird ein Verfahren zur Herstellung von Diarylketonen durch Umsetzung von aromatischen Verbindungen zu Diarylalkanen und Oxydation der Diarylalkane zu den entsprechenden Diarylketonen, beschrieben, bei dem man eine aromatische Verbindung mit einer vinylaromatischen Verbindung, besonders Styrol in Gegenwart eines stark sauren, großporigen Zeoliths als Katalysator zum 1,1-Diarylethan alkyliert und das erhaltene 1,1-Diarylethan in der Gasphase katalytisch zum Diarylketon oxydiert. Das Verfahren hat den Vorteil, daß dabei kein Chlorwasserstoff als Nebenprodukt anfällt.

EP 0 860 418 A1

**Beschreibung**

Die Erfindung betrifft ein Verfahren zur Herstellung aromatischer Ketone, insbesondere von Diarylketonen, durch Umsetzung von aromatischen Verbindungen zu Diarylalkanen und Oxydation der Diarylalkane zu den entsprechenden Diarylketonen.

Es ist bekannt, aromatische Ketone durch Acylierung von aromatischen Verbindungen nach Friedel-Crafts herzustellen. Dies erfordert den Einsatz von aktivierten Carbonsäurederivaten, insbesondere von Carbonsäurechloriden, und stöchiometrischen oder höheren Mengen an Katalysator, insbesondere $AlCl_3$, der nach der Reaktion neutralisiert und als Salzfracht entsorgt werden muß. Bei besonders reaktiven Aromaten kann man zwar auch Katalysatoren einsetzen, die nicht in stöchiometrischer Menge verbraucht werden, z.B. sulfatisiertes Zirkondioxid ($ZrO_2$-$SO_4$) oder $Fe_2O_3$. Trotzdem bleibt das Problem, daß bei der Acylierung nach Friedel-Crafts HCl frei wird, was zum einen korrosionsbeständige (emaillierte) Apparate erfordert und zum anderen eine aufwendige Abgaswäsche notwendig macht. Darüber hinaus ist die Herstellung aromatischer Säurechloride aufwendig und relativ teuer.

Ein anderes Verfahren zur Herstellung aromatischer Ketone besteht darin, daß aromatische Kohlenwasserstoffe mit Benzylchloriden zu Diarylmethanen alkyliert und anschließend zum Diarylketon oxydiert werden. Auch bei diesem Verfahren wird Chlorwasserstoff frei, der entsorgt werden muß.

Aufgabe der Erfindung war es, ein Verfahren zur Herstellung von Diarylketonen vorzuschlagen, bei dem kein Chlorwasserstoff als Nebenprodukt entsteht.

Die Erfindung geht aus von einem Verfahren zur Herstellung von Diarylketonen durch Kondensation von aromatischen Verbindungen zu Diarylalkanen und Oxydation der Diarylalkane zu den entsprechenden Diarylketonen.

Das erfindungsgemäße Verfahren ist dadurch gekennzeichnet, daß man eine aromatische Verbindung mit einer vinylaromatischen Verbindung in Gegenwart eines stark sauren, großporigen Zeoliths als Katalysator zum 1,1-Diarylethan alkyliert und das erhaltene 1,1-Diarylethan in der Gasphase katalytisch zum Diarylketon oxydiert.

Als vinylaromatische Verbindung wird vorzugsweise Styrol oder ein substituiertes, insbesondere kernsubstituiertes Styrol, z.B. p-Chlorstyrol, o-Fluorstyrol, p- oder m- Methylstyrol, o-, m- oder p-Vinylanisol oder p-Vinylbiphenyl, eingesetzt.

Als zu alkylierende aromatische Verbindung wird vorzugsweise ein aromatischer Kohlenwasserstoff verwendet, der gegebenenfalls durch niedere Alkylgruppen, z.B. solche mit 1 bis 4 Kohlenstoffatomen, substituiert sein kann. Die aromatische Verbindung kann ein- oder mehrkernig sein. Einkernige Aromaten werden bevorzugt, doch können auch mehrkernige, z.B. Naphthalin oder entsprechend substituierte Naphthaline, zum Einsatz kommen, soweit sich die erhaltenen Diarylethane in der zweiten Stufe noch ohne Probleme in der Gasphase oxydieren lassen. Als aromatische Verbindungen können auch Kohlenwasserstoffe eingesetzt werden, die in inerte Substituenten, wie Halogenatome oder Alkoxygruppen, insbesondere Methoxygruppen, tragen.

Die Oxydation der 1,1-Diarylethane erfolgt in Gegenwart eines Katalysators, der als Aktivkomponente eine Verbindung eines Elements der V. bis VIII. Nebengruppe des Periodensystems, gegebenenfalls auf einen Träger aus einem Oxid eines Elements der III. oder IV. Hauptgruppe oder der IV. Nebengruppe des Periodensystems, enthält. Als Aktivkomponenten kommen insbesondere Oxide der genannten Elemente, z.B. $V_2O_5$ oder $Sb_2O_3$, und als Träger z.B. $TiO_2$ in Betracht. Der Katalysator kann dann auf Formkörper, z.B. Kugeln oder Monolithe aus keramischem Material, z.B. aus einem Silikat, aufgebracht werden. Die Reaktionstemperatur liegt im allgemeinen im Bereich von 300 bis 450, vorzugsweise von 320 bis 380°C.

Die erste Stufe der Umsetzung, die Alkylierung, ist als solche bekannt und z.B. in der EP-A 742 190 oder der DE-A 195 25 682 beschrieben. Auf die Ausführungen in diesen Druckschriften wird hier verwiesen.

Die Alkylierung von aromatischen Verbindungen, besonders Kohlenwasserstoffen, mit vinylaromatischen Verbindungen kann bevorzugt in flüssiger Phase durchgeführt werden. Die Reaktionstemperatur liegt im Bereich von 10 bis 280°C, besonders bevorzugt im Bereich von 80 bis 200°C. Als Katalysatoren können stark saure, großporige Zeolithe, insbesondere 12-Ring-Zeolithe, z.B. vom Typ Beta-Zeolith (BEA), Y-Zeolith oder Mordenit, eingesetzt werden. Der Katalysator kann entweder in Pulverform im Reaktionssystem suspendiert oder als Formkörper in einem Festbettreaktor eingesetzt werden.

Die Reaktion wird bevorzugt unter Bedingungen durchgeführt, die nur eine geringe Konzentration von Styrol zulassen. Dies kann durch den Einsatz von verdünnten Styrol-Lösungen oder durch langsames Zudosieren des Styrols erfolgen. Eine besonders bevorzugte Methode zur diskontinuierlichen Herstellung besteht darin, daß man den aromatischen Kohlenwasserstoff zusammen mit dem darin suspendierten Katalysator in einem Rührkessel vorlegt und die venylaromatische Verbindung (oder ihre Lösung) während der Reaktion so langsam zutropft, daß sich ein Fließgleichgewicht zwischen der zudosierten und der abreagierenden Vinylverbindung auf einem niedrigen Konzentrationsniveau einstellt. Die Konzentration an Vinylverbindung, besonders Styrol, sollte 3 %, bevorzugt 0,1 %, besonders bevorzugt 0,05 % nicht überschreiten.

Analog dazu besteht eine besondere Methode zur kontinuierlichen Herstellung darin, daß man einen Rührkessel mit Überlauf verwendet, den Katalysator als Suspension im jeweiligen Aromaten im Rührkessel vorlegt und anschließend kontinuierlich ein Vinylaromat/Aromat-Gemisch so zudosiert, daß sich die vorstehend angegebene niedrige stationäre Konzentration an Vinylaromaten einstellt. Das am Überlauf austretende Reaktionsgemisch kann mit einem Settler oder einem Filter vom Katalysator abgetrennt werden. Der Katalysator kann, gegebenenfalls nach einer Regenerierung, in das Reäktionsgefäß zurückgeführt werden.

Anstelle des Rührkessels kann man auch einen mit Katalysatorformkörpern gefüllten Rohrreaktor verwenden, wenn man durch intensive Produktrückführung für eine gute Durchmischung sorgt.

Die Oxidation der 1,1-Diarylethane wird heterogen-katalytisch in der Gasphase durchgeführt. Die Reaktion wird bevorzugt in Festbettreaktoren durchgeführt, wobei die Reaktionsbedingungen und eingesetzten Katalysatoren überraschenderweise die gleichen sind, wie sie für die katalytische Oxidation von o-Xylol zu Phthalsäureanhydrid oder von Durol zu Pyromellitsäureanhydrid typisch sind. Insbesondere können Katalysatoren eingesetzt werden wie sie in dem DE-A 40 13 051, 30 60 586, und 3 30 600 94 beschrieben sind. Die in den genannten Patentveröffentlichungen beschriebenen Katalysatoren wurden speziell zur Herstellung von Carbonsäuren bzw. deren Anhydriden entwickelt. Die Eignung zur Herstellung von aromatischen Ketonen aus 1,1-Diarylethanen war bisher nicht bekannt und auch nicht voraussehbar.

Zur Analyse der nachfolgend beschriebenen Produkte wurde ein Gaschromatograph mit einer 30 m langen Standard-Kapillarsäule verwendet. (Temperaturprogramm: 5 min bei 60°C; dann Steigerung um 10°C/min bis 300°C; 15 min halten bei der Endtemperatur). Die Ermittlung der Mengen der Produkte erfolgte durch Vergleich der Peaks mit dem von Toluol, das dem Gemisch in einem Anteil von 10 % zugesetzt wurde, als innerem Standard.

Die Mengen der drei entstandenen Dimeren, die durch Gaschromatographie/Massenspektroskopie (GC/MS) ermittelt wurden, sind in der folgenden Zusammenstellung zusammengezahlt worden. Ihr Eichfaktor wurde mit dem für Diphenylethan gleichgesetzt. Die Eichfaktoren für Benzol (nicht aufgelistet), Styrol und Diphenylethan wurden über eine Konzentrationsreihe mit Toluol als innerem Standard bestimmt.

**Beispiel**

In einen 1 l fassenden Autoklaven mit Magnetrührer wurden in einem Katalysatorkorb 150 g Katalysator (Beta-Zeolith, mit 20 % Aluminiumoxidhydrat zu 1,5 mm dicken Strängen verformt) und 500 ml Benzol vorgelegt. Es wurde auf 200°C erwärmt mit anschließend kontinuierlich eine Mischung aus 10 % Styrol und 90 % Benzol mit einer Rate von 1 l/h zudosiert. Das Produkt wurde kontinuierlich über ein Überströmventil (Druck = 15 bar) entnommen. Der Versuch wurde 800 Stunden lang gefahren und das erhaltene Produktgemisch durch Destillieren aufgearbeitet.

Dazu wurden bei 80°C und ca. 1 bar das Benzol und bei 135°C und ca. 10 mbar das 1,1-Diphenylethan abdestilliert. Aus dem verbleibenden hochsiedenden Rückstand wurden bei 128°C und ca. 2 mbar die Styroldimeren (hauptsächlich 1-Methyl-3-phenyl-indan) abgetrennt und bei 171°C und ca. 1 mbar das 1,2-Bis-($\alpha$-methyl-benzyl)benzol und bei 179°C und ca. 1 mbar das 1,4-Bis-($\alpha$-methyl-benzyl)benzol durch franktionierte Destillation gewonnen.

Die Gleichungen gehen wie folgt:

$$Ph\text{-}CH=CH_2+PhH \rightarrow Ph\text{-}CH(CH_3)\text{-}Ph$$

$$Ph\text{-}CH=CH_2+Ph\text{-}CH(CH_3)\text{-}Ph \rightarrow Ph\text{-}CH(CH_3)\text{-}1,4\text{-}Phenylen\text{-}CH(CH_3)\text{-}Ph +Ph\text{-}CH(CH_3)\text{-}1,2\text{-}Phenylen\text{-}CH(CH_3)\text{-}Ph$$

In den nachfolgenden Oxydationsversuchen wurden das 1,1-Diphenylethan, das 1,2-Bis-($\alpha$-methyl-benzyl)benzol und das 1,4-Bis-($\alpha$-methyl-benzyl)benzol zu den entsprechenden aromatischen Ketonen oxydiert.

$$Ph\text{-}CH(CH_3)\text{-}Ph \rightarrow Ph\text{-}CO\text{-}Ph$$

$$Ph\text{-}CH(CH_3)\text{-}Phenylen\text{-}CH(CH_3)\text{-}Ph \rightarrow PH\text{-}CO\text{-}Phenylen\text{-}CO\text{-}Ph$$

**Herstellung des Oxydationskatalysators**

700 g Steatitkugeln (Magnesiumsilikat) mit einem Durchmesser von 3-4 mm wurden in einer Dragiertrommel auf 210°C erhitzt und mit einer Suspension aus 400 g Anatas (TiO$_2$) mit einer Oberfläche nach Brunauer, Emmet und Teller (BET-Methode; J. Am. Chem. Soc. 60(1938), 309 ff), von 21 m$^2$/g, 57,6 g Vanadyloxalat, 14,4 g Antimontrioxid, 3,3 g Ammoniumdihydrogenphosphat, 2,6 g Cäsiumsulfat, 618 g Wasser und 128 g Formamid besprüht, bis das Gewicht der aufgetragenen getrockneten Schicht 10,5 % des Gesamtgewichts des fertigen Katalysators betrug. Die auf diese Weise aufgebrachte katalytisch aktive Masse, also die Katalysatorschale, enthielt 0,2 Gew.-% Phosphor (berechnet als P), 7,5 Gew.-% Vanadium (berechnet als V$_2$O$_5$), 3,2 Gew.-% Antimon (berechnet als Sb$_2$O$_3$), 0,4 Gew.-% Cäsium (berechnet als Cs) und 88,75 Gew.-% Titandioxid.

**Oxydation**

85 g des obigen Katalysator wurden in ein 60 cm langes Eisenrohr mit einer lichten Weite von 20 mm eingefüllt. Das Eisenrohr wurde elektrisch temperiert, eine Thermohülse von 4 mm Durchmesser mit eingebautem Zugelement diente der Katalysatortemperaturmessung. Durch das Rohr wurden stündlich von oben nach unten 330 Nl (Normliter) Luft mit Beladungen an Diarylethanen von 0,4 Vol. % geleitet. Dabei wurden bei einer Reaktortemperatur von 350°C die in folgender Tabelle zusammengefaßten Ergebnisse erhalten.

Tabelle

| Oxydation von Diarylethanen an V/Sb/Ti-Katalysatoren | | | |
|---|---|---|---|
| Edukt | Umsatz (bezogen auf Edukt) | Ausbeute an $CO_x$ (CO + $CO_2$) | Wertprodukt-Ausbeuten |
| 1,1-Diphenylethan | 92 % | 30 % | 48 % Benzophenon 15 % Benzoesäure 3 % Benzaldehyd |
| 1,2-Bis-($\alpha$-methyl benzyl)benzol | 80 % | 38 % | 30 % Diketon 8,5 % Monoketon 15 % Benzoesäure 3 % Benzaldehyd |
| 1,4-Bis-($\alpha$-methyl benzyl)benzol | 94 % | 37 % | 40 % Diketon 3 % Monoketon 14 % Benzoesäure 2 % Benzaldehyd |

**Patentansprüche**

1. Verfahren zur Herstellung von Diarylketonen durch Umsetzung von aromatischen Verbindungen zu Diarylalkanen und Oxydation der Diarylalkane zu den entsprechenden Diarylketonen, dadurch gekennzeichnet, daß man eine aromatische Verbindung mit einer vinylaromatischen Verbindung in Gegenwart eines stark sauren, großporigen Zeoliths als Katalysator zum 1,1-Diarylethan alkyliert und das erhaltene 1,1-Diarylethan in der Gasphase katalytisch zum Diarylketon oxydiert.

2. Verfahren nach Anspruch 1, dadurch gekennzeichnet, daß man als vinylaromatische Verbindung Styrol oder ein substituiertes Styrol einsetzt.

3. Verfahren nach Anspruch 1 oder 2, dadurch gekennzeichnet, daß man als aromatische Verbindung, die mit der vinylaromatischen Verbindung alkyliert wird, eine einkernige aromatische Verbindung einsetzt.

4. Verfahren nach Anspruch 3, dadurch gekennzeichnet, daß man als einkernige aromatische Verbindung einen aromatischen Kohlenwasserstoff einsetzt.

5. Verfahren nach Anspruch 1, dadurch gekennzeichnet, daß man als stark sauren, großporigen Zeolith einen 12-Ring-Zeolith einsetzt.

6. Verfahren nach Anspruch 5, dadurch gekennzeichnet, daß der 12-Ring-Zeolith ein Beta- oder y-Zeolith oder ein Mordenit ist.

7. Verfahren nach Anspruch 1, dadurch gekennzeichnet, daß man die Oxydation in Gegenwart eines Katalysators durchführt, der als Aktivkomponente eine Verbindung eines Elements der V. bis VIII. Nebengruppe des Periodensystems, gegebenenfalls auf einem Träger aus einem Oxid eines Elements der III. oder IV. Hauptgruppe oder der IV. Nebengruppe des Periodensystems, enthält.

8. Verfahren nach Anspruch 7, dadurch gekennzeichnet, daß der Katalysator auf einen Formkörper aus keramischem Material aufgebracht ist.

9. Verfahren nach Anspruch 1, dadurch gekennzeichnet, daß die Alkylierung in flüssiger Phase bei einer Temperatur im Bereich von 10 bis 280°C durchgeführt wird.

10. Verfahren nach Anspruch 9, dadurch gekennzeichnet, daß die Alkylierung in einer Lösung durchgeführt wird, die nicht mehr als 3 % an vinylaromatischer Verbindung enthält.

11. Verfahren nach Anspruch 1, dadurch gekennzeichnet, daß man die Oxydation in einem Festbettreaktor durchführt.

# EP 0 860 418 A1

**Europäisches Patentamt**

## EUROPÄISCHER RECHERCHENBERICHT

Nummer der Anmeldung

EP 98 10 2707

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| Y | EP 0 313 661 A (NIPPON PETROCHEMICALS CO LTD) 3.Mai 1989 * Ansprüche * --- | 1-11 | C07C45/36 C07C49/784 C07C49/786 C07C2/66 C07C15/16 |
| Y | DATABASE WPI Section Ch, Week 8622 Derwent Publications Ltd., London, GB; Class A60, AN 86-141956 XP002067225 & JP 61 078 747 A (NIPPON SHOKUBAI KAGAKU KOGYO CO LTD) * Zusammenfassung * --- | 1-11 | |
| Y | US 4 299 987 A (DOLHYJ SERGE R ET AL) 10.November 1981 * das ganze Dokument * --- | 1-11 | |
| D,Y | EP 0 753 498 A (BASF AG) 15.Januar 1997 * Ansprüche * --- | 1-11 | |
| D,Y | EP 0 742 190 A (BASF AG) 13.November 1996 * Ansprüche * ----- | 1-11 | RECHERCHIERTE SACHGEBIETE (Int.Cl.6) C07C |

Der vorliegende Recherchenbericht wurde für alle Patentansprüche erstellt

| Recherchenort | Abschlußdatum der Recherche | Prüfer |
|---|---|---|
| DEN HAAG | 8.Juni 1998 | Bonnevalle, E |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

6